# EUROPEAN PATENT SPECIFICATION

(11) **EP 4 288 420 B1**
(45) Date of publication and mention of the grant of the patent: **09.07.2025**
(21) Application number: 22702663.0
(22) Date of filing: 26.01.2022
(51) Int. Cl.: C07D 301/12, C07D 301/32

(54) **PROCESS FOR THE EPOXIDATION OF PROPENE**
VERFAHREN ZUR EPOXIDIERUNG VON PROPEN
PROCÉDÉ D'ÉPOXYDATION DE PROPÈNE

(30) Priority: 03.02.2021 EP 21154894
(43) Date of publication of application: 13.12.2023
(73) Proprietor: Evonik Operations GmbH, 45128 Essen (DE); thyssenkrupp Uhde GmbH, 44141 Dortmund (DE)
(72) Inventor: BERNHARD, Maik, 01471 Radeburg (DE); GÖTZ, Sören, 63796 Kahl am Main (DE); BRENDEL, Marc, 63486 Bruchköbel (DE); ANTON, Johan, 46284 Dorsten (DE); PASCALY, Matthias, 48163 Münster (DE); NOLL, Thomas, 63755 Alzenau (DE); SCHEMEL, Jürgen, 60388 Frankfurt (DE)
(74) Representative: Evonik Patent Association
(86) International application number: PCT/EP2022/051741
(87) International publication number: WO 2022/167284

(56) References cited:
- WO-A1-2018/206505

## Description

### Field of the invention

The present invention relates to a process for the epoxidation of propene with hydrogen peroxide in the presence of a titanium silicalite catalyst.

### Background of the invention

The epoxidation of propene with hydrogen peroxide in the presence of a titanium silicalite catalyst is known from EP 0 100 119 A1. The reaction of propene with hydrogen peroxide in the presence of a titanium zeolite catalyst is usually carried out in a methanol solvent to achieve high reaction rates and product selectivity. In addition to propene oxide, the epoxidation reaction produces byproducts, such as formaldehyde, acetaldehyde.

By-products acetaldehyde and propionaldehyde are difficult to separate from the propene oxide product. WO 2004/048355 discloses a method for removing methanol and acetaldehyde from a crude propene oxide in a single distillation column by an extractive distillation where a compound containing an unsubstituted NH₂ group and capable of reacting with acetaldehyde at the conditions of distillation is additionally fed at or above the feeding point of the crude propene oxide. An aqueous hydrazine solution is preferably used as the additionally fed compound. Water is particularly preferred as the extraction solvent. The method provides propene oxide of high purity suitable for making polyether polyols.

WO 2004/048354 teaches recovery of a solvent stream from the reaction mixture of an epoxidation of an olefin with hydrogen peroxide in the presence of a titanium-containing zeolite catalyst, where the recovered solvent stream is treated to contain less than 50 wppm nitrogen in the form of organic nitrogen compounds before it is recycled to the epoxidation step, in order to reduce deactivation of the catalyst upon recycling of the solvent. The solvent treatment is preferably an acid treatment. WO 2004/048354 teaches that the acid treatment can be carried out by adding a carboxylic acid or a mineral acid to the solvent stream before or during a distillation recovering the solvent as an overhead product or by treating an overhead product obtained by distillation with an acidic ion exchanger.

WO 2018/206505 teaches recovery of a methanol stream from the reaction mixture of an epoxidation of an olefin with hydrogen peroxide in the presence of a titanium-containing zeolite catalyst, where a mixture comprising methanol, water and peroxides is subjected to a catalytic hydrogenation; the hydrogenated solvent is separated in at least one distillation stage, adding an acid to the hydrogenated solvent mixture or to at least one distillation stage, to provide a recovered methanol as an overhead product; the recovered methanol is passed through a bed of an acidic ion exchange resin to provide a treated methanol; and the treated methanol is recycled to the epoxidation reaction. The document suggests using two ion exchanger beds arranged in parallel to allow regeneration of the ion exchange resin without interrupting the methanol treatment.

### Summary of the invention

The inventors of the present invention have observed that the steps of methanol recovery and recycling disclosed in WO 2018/206505, although effective for maintaining long term activity of the epoxidation catalyst, may lead to breakage of the catalyst in a process using a shaped titanium zeolite epoxidation catalyst in a fixed bed reactor. The inventors have also observed that such catalyst breakage can occur shortly after a bed of an acidic ion exchange resin, used to treat the methanol before recycling it to the reaction step, has been regenerated. The inventors have found that catalyst breakage can be reduced or avoided by washing the regenerated bed of an acidic ion exchange resin with methanol until the methanol exiting the resin bed has an apparent pH higher than 2 before methanol that has been treated with the acidic ion exchange resin is recycled to the reaction step.

Subject of the invention is therefore a process for the epoxidation of propene which comprises the steps a) to e):
a) reacting propene with hydrogen peroxide in the presence of a methanol solvent and a shaped titanium zeolite epoxidation catalyst in a fixed bed reactor to provide a reaction mixture,
b) separating from the reaction mixture of step a) a crude propene oxide and a solvent mixture comprising methanol and water,
c) separating the solvent mixture of step b) in at least one distillation stage, providing a recovered methanol as an overhead product,
d) passing the recovered methanol of step c) through a bed of an acidic ion exchange resin, providing a treated methanol, and
e) recycling the treated methanol of step d) as methanol solvent to step a),
wherein the bed of an acidic ion exchange resin is periodically regenerated by passing a solution of a regenerating acid through the resin bed to provide a regenerated bed of ion exchange resin and the regenerated bed of ion exchange resin is washed by passing methanol through the regenerated bed of ion exchange resin until the methanol exiting the resin bed has an apparent pH higher than 2.0 before the regenerated bed of ion exchange resin is reused in step d).

### Detailed description of the invention

In step a) of the process of the invention, propene is reacted with hydrogen peroxide in the presence of a methanol solvent and a shaped titanium zeolite epoxidation catalyst in a fixed bed reactor to provide a reaction mixture.

Propene is preferably used in a molar excess to hydrogen peroxide, preferably at a molar ratio of propene to hydrogen peroxide of from 1.1:1 to 30:1, more preferably 2:1 to 10:1 and most preferably 3:1 to 5:1. In a preferred embodiment, propene is used in an excess sufficient to maintain an additional liquid phase rich in propene throughout step a). The propene may contain propane, preferably with a molar ratio of propane to propene of from 0.001 to 0.20 and more preferably of from 0.08 to 0.12.

Hydrogen peroxide can be used as an aqueous solution, preferably containing from 30 to 75 % by weight hydrogen peroxide and most preferably from 40 to 70 % by weight. The aqueous hydrogen peroxide solution is preferably made by an anthraquinone process.

The reaction of propene with hydrogen peroxide is carried out in the presence of a methanol solvent. The methanol solvent can be a technical grade methanol, a solvent stream recovered in the work-up of the epoxidation reaction mixture or a mixture of both. The methanol solvent may comprise other solvents in minor amounts, such as ethanol, with the amount of such other solvents preferably being less than 2 % by weight. The methanol solvent may also comprise water, preferably from 2 to 13 % by weight water. The solvent is preferably used in the epoxidation in a weight ratio of 0.5 to 20 relative to the combined weight of water and hydrogen peroxide.

The shaped titanium zeolite epoxidation catalyst used in step a) preferably comprises a titanium zeolite containing titanium atoms on silicon lattice positions. Preferably, a titanium silicalite catalyst is used, preferably with an MFI or MEL crystal structure. Most preferably a titanium silicalite-1 catalyst with MFI structure as known from EP 0 100 119 A1, is used. The shaped titanium silicalite catalyst is preferably employed in the form of granules, extrudates or shaped bodies. Extrudates with a diameter of 1 to 5 mm are preferably used. For the shaping process the catalyst may contain 1 to 99% of a binder or carrier material, all binders and carrier materials being suitable that do not react with hydrogen peroxide or with propene oxide under the reaction conditions employed for the epoxidation, silica being preferred as binder. The amount of catalyst employed may be varied within wide limits and is preferably chosen so that a hydrogen peroxide consumption of more than 90%, preferably more than 95%, is achieved within 1 minute to 5 hours under the employed epoxidation reaction conditions.

The epoxidation reaction of step a) is preferably carried out at a temperature of 20 to 80°C, more preferably at 25 to 60°C. The epoxidation reaction is preferably carried out at a pressure that is higher than the vapor pressure of propene at the reaction temperature in order to maintain the propene dissolved in the solvent or present as a separate liquid phase. The pressure in step a) is preferably from 1.9 to 5.0 MPa, more preferably 2.1 to 3.6 MPa and most preferably 2.4 to 2.8 MPa. Using an excess of propene at a high pressure provides high reaction rate and hydrogen peroxide conversion and at the same time high selectivity for propene oxide.

The epoxidation reaction is preferably carried out with addition of ammonia to improve epoxide selectivity as described in EP 0 230 949 A2. Ammonia is preferably added with a weight ratio of ammonia to the initial amount of hydrogen peroxide of from 0.0001 to 0.003.

The epoxidation reaction of step a) is carried out in a fixed bed reactor, preferably by passing a mixture comprising propene, hydrogen peroxide and methanol solvent through a fixed bed comprising the shaped titanium zeolite catalyst. The fixed bed reactor is preferably a tube bundle reactor and the catalyst fixed bed is arranged inside the reactor tubes. The fixed bed reactor is preferably equipped with cooling means and cooled with a liquid cooling medium. In a preferred embodiment, a tube bundle reactor as described in WO 2017/089076 is used and the reactor is cooled as described in this document. The temperature profile along the length of the catalyst fixed bed is preferably adjusted to keep the reaction temperature along 70 to 98 %, preferably along 80 to 95 %, of the length of the catalyst fixed bed within a range of less than 5 °C, preferably within a range of from 0.5 to 3 °C. The temperature of the cooling medium fed to the cooling means is preferably adjusted to a value 3 to 13 °C lower than the maximum temperature in the catalyst fixed bed. The epoxidation reaction mixture is preferably passed through the catalyst bed in down flow mode, preferably with a superficial velocity from 1 to 100 m/h, more preferably 5 to 50 m/h, most preferred 5 to 30 m/h. The superficial velocity is defined as the ratio of volume flow rate/cross section of the catalyst bed. Additionally it is preferred to pass the reaction mixture through the catalyst bed with a liquid hourly space velocity (LHSV) from 1 to 20 h⁻¹, preferably 1.3 to 15 h⁻¹. It is particularly preferred to maintain the catalyst bed in a trickle bed state during the epoxidation reaction. Suitable conditions for maintaining the trickle bed state during the epoxidation reaction are disclosed in WO 02/085873 on page 8 line 23 to page 9 line 15. The epoxidation reaction is most preferably carried out with a catalyst fixed bed maintained in a trickle bed state at a pressure close to the vapor pressure of propene at the reaction temperature, using an excess of propene that provides a reaction mixture comprising two liquid phases, a solvent rich phase and a propene rich liquid phase. Two or more fixed bed reactors may be operated in parallel or in series in order to be able to operate the epoxidation process continuously when regenerating the epoxidation catalyst. Regeneration of the epoxidation catalyst can be carried out by calcination, by treatment with a heated gas, preferably an oxygen containing gas or by a solvent wash, preferably by the periodic regeneration described in WO 2005/000827. Regeneration of the epoxidation catalyst is preferably carried out without removing it from the fixed bed reactor. Different methods of regeneration may be combined.

The epoxidation reaction of step a) provides a reaction mixture comprising propene oxide formed by the reaction, methanol and water. When propene is used in a molar excess to hydrogen peroxide, the reaction mixture also comprises unreacted propene. The reaction mixture may contain unreacted hydrogen peroxide and by-products formed from propene oxide, such as 1,2-propanediol, 1-methoxy-2-propanol, 2-methoxy-1-propanol, 1-hydroperoxy-2-propanol and 2-hydroperoxy-1-propanol.

In step b) of the process of the invention, a crude propene oxide is separated from the reaction mixture of step a) and a solvent mixture comprising methanol and water is separated from the reaction mixture of step a). The separation of the crude propene oxide and the solvent mixture from the reaction mixture can be carried out by methods known from the prior art. The separation of the solvent mixture from the reaction mixture is preferably carried out to provide a solvent mixture which comprises less than 5 % by weight propene and less than 2 % by weight propene oxide. The separation is preferably carried out to provide a crude propene oxide comprising from 15 to 97 % by weight propene oxide and from 2 to 84 % by weight methanol.

Preferably, in step b) the reaction mixture is subjected to a pressure reduction and propene vapor formed by the pressure reduction is recompressed and cooled to recover propene by condensation. The compressed propene vapor is preferably fed to a propene distillation column and separated into an overhead product comprising non-reacted propene and a bottoms product containing compounds having a boiling point higher than propene, such as propene oxide and methanol solvent. The overhead product comprising non-reacted propene can be recycled to the epoxidation reaction. The bottoms product can be combined with the liquid mixture remaining after the pressure reduction. The liquid mixture remaining after the pressure reduction is preferably separated by distillation in a pre-separation column to provide a crude propene oxide comprising propene oxide, methanol and residual propene as an overhead product and a solvent mixture comprising methanol and water as a bottoms product. The pre-separation column is preferably operated to provide an overhead product comprising from 20 to 60 % of the methanol contained in the liquid phase of the last pressure reduction step. The pre-separation column preferably has from 5 to 20 theoretical separation stages in the stripping section and less than 3 theoretical separation stages in a rectifying section and is most preferably operated without reflux and without a rectifying section to minimize the residence time of propene oxide in the pre-separation column. The pre-separation column is preferably operated at a pressure of from 0.16 to 0.3 MPa. Propene oxide and methanol are condensed from the overhead product of the pre-separation column and propene is preferably stripped from the resulting condensate in a propene stripping column which provides a bottom stream comprising propene oxide and methanol which is essentially free of propene.

In step c) of the process of the invention, the solvent mixture of step b) is separated in at least one distillation stage to provide a recovered methanol as an overhead product. The solvent mixture is preferably separated in two subsequent distillation stages providing recovered methanol as an overhead product from both stages. The two distillation stages are preferably operated with a higher pressure in the second stage and overhead product vapor from the second stage is used for heating the bottoms evaporator of the first stage in order to save energy.

In step d) of the process of the invention, the recovered methanol of step c) is passed through a bed of an acidic ion exchange resin to provide a treated methanol. Both strongly acidic ion exchange resins and weakly acidic ion exchange resins may be used. Preferred are strongly acidic ion exchange resins containing SO₃H groups and weakly acidic ion exchange resins containing COOH groups. Most preferred are strongly acidic ion exchange resins containing sulfonic acid groups. The acidic ion exchange resin is preferably based on an organic polymer, such as crosslinked polystyrene, or an organic inorganic hybrid polymer, such as a polysiloxane. The acidic ion exchange resin may be a gel type solid or a macroporous solid. Preferably, two ion exchanger beds are arranged in parallel to allow regeneration of the ion exchange resin without interrupting the methanol treatment. Preferably, the apparent pH of the treated methanol is monitored and the acidic ion exchange resin is regenerated when the apparent pH of the treated methanol exceeds a threshold value selected in the range of from 4 to 7. Especially the acidic ion exchange resin is regenerated when the apparent pH is 2-4 units higher than the apparent pH of the treated methanol obtained with a fresh regenerated acidic ion exchange resin after washing and adjustment of a steady state of the apparent pH. Thetreated methanol provided in step d) contains less basic impurities such as ammonia and organic amines and will in general contain acetaldehyde at a lower concentration than the recovered methanol of step c) and 1,1-dimethoxyethane at a higher concentration due to acetalisation of acetaldehyde with methanol catalyzed by the acidic ion exchange resin.

In step e) of the process of the invention, the treated methanol of step d) is recycled as methanol solvent to step a). Steps a) to e) of the process of the invention are preferably carried out continuously, preferably using a continuously operated reactor in step a) and rectification columns in separation steps b) and c).

In a preferred embodiment, an acid is added to at least one distillation stage of step c) or to the solvent mixture obtained in step b) before separating it in step c). When the acid is added to a distillation stage, it is preferably added at a feed point above the feed point for the solvent mixture and below the column top. The acid may also be added to the reflux stream of the distillation column. Preferably, the acid is added to the solvent mixture obtained in step b) before separating it in step c). Adding an acid in step c) reduces the content of volatile organic amines in the recovered methanol, extends the time span for which the bed of an acidic ion exchange resin can be used in step d) before it has to be regenerated and prevents deactivation of the epoxidation catalyst by organic amines recycled to step a) with the recovered methanol. The acid is preferably added in an amount providing a content of less than 250 ppm by weight nitrogen in the form of organic nitrogen compounds in the recovered methanol, more preferably in an amount providing a content of less than 50 ppm by weight nitrogen in the form of organic nitrogen compounds. The acid may be a mineral acid, such as nitric acid, sulfuric acid, hydrochloric acid, phosphoric acid or perchloric acid; a sulfonic acid, such as methane sulfonic acid; or a carboxylic acid, such as formic acid, acetic acid, propionic acid, butyric acid, pentanoic acid, hexanoic acid, heptanoic acid, octanoic acid, nonanoic acid, decanoic acid, undecanoic acid, dodecanoic acid, oxalic acid, malonic acid, succinic acid, glutaric acid, adipic acid, maleic acid or fumaric acid. Preferred are sulfuric acid and phosphoric acid, most preferred is sulfuric acid. The amount of nitrogen in the form of organic nitrogen compounds can be determined as the difference between the total amount of nitrogen and the amount of nitrogen in the form of inorganic nitrogen compounds. The total amount of nitrogen can be determined by the Kjeldahl method as described in DIN 53625. The recovered methanol will usually contain no inorganic compounds other than ammonia and the amount of nitrogen in the form of inorganic nitrogen compounds may therefore be determined by ion chromatography of an acidified sample detecting ammonium ions. The acid is preferably added in an amount providing an apparent pH of from 1.6 to 5.0, more preferably from 1.8 to 4.0, in the bottoms product remaining after recovery of methanol. The term apparent pH refers to the value measured at 20 °C with a pH meter equipped with a pH sensitive glass electrode calibrated with aqueous buffer solutions. Maintaining the apparent pH within these ranges provides a recovered methanol with a low content of alkyl amines and at the same time reduces or prevents acid corrosion of the distillation equipment.

In another preferred embodiment, which may be combined with the embodiment described in the preceding paragraph, the solvent mixture separated in step b) is subjected to a catalytic hydrogenation before it is separated in step c). The catalytic hydrogenation is preferably carried out at a hydrogen partial pressure of from 0.5 to 30 MPa, more preferably of from 1 to 25 MPa and most preferably of from 1 to 5 MPa. The temperature is preferably in the range of from 80 to 180 °C, more preferably from 90 to 150 °C. The catalytic hydrogenation is carried out in the presence of a hydrogenation catalyst, preferably a heterogeneous hydrogenation catalyst. Raney nickel and Raney cobalt may be used as hydrogenation catalyst. Preferably, a supported metal catalyst comprising one or more of metals selected from the group consisting of Ru, Rh, Pd, Pt, Ag, Ir, Fe, Cu, Ni and Co on a catalyst support is used. The metal is preferably platinum, palladium, iridium, ruthenium or nickel and most preferably ruthenium or nickel. The catalyst support can be any solid which is inert and does not deteriorate under the hydrogenation conditions. Suitable as catalyst support are activated carbon, the oxides SiO₂, TiO₂, ZrO₂ and Al₂O₃, and mixed oxides comprising at least two of silicon, aluminum, titanium and zirconium. SiO₂, Al₂O₃ and mixed oxides of silicon and aluminum are preferably used as the catalyst support for the supported metal catalyst. The catalyst support is preferably shaped as spheres, pellets, tablets, granules or extrudates. Preferred are extrudates with a diameter of from 0.5 to 5mm, especially from 1 to 3 mm, and a length of from 1 to 10 mm. The supported metal catalyst preferably comprises from 0.01 to 60 wt.% metal. Supported noble metal catalysts preferably comprise from 0.1 to 5 % metal. Supported nickel and cobalt catalysts preferably comprise from 10 to 60 % metal. The supported metal catalyst may be prepared by methods known in the art, preferably by impregnating the catalyst support with a metal salt followed by reducing the metal salt to the catalytically active metal. Suitable supported metal catalyst are commercially available, for example from Clariant under the NISAT^{®} trade name and from Evonik Industries under the Noblyst^{®} trade name. The catalytic hydrogenation converts unreacted hydrogen peroxide to water and the by-product peroxides 1-hydroperoxy-2-propanol and 2-hydroperoxy-1-propanol formed in step a) to 1,2-propanediol and prevents by-product formation by peroxide decomposition in subsequent work-up stages. The catalytic hydrogenation is preferably carried out to a conversion of hydrogen peroxide that provides a hydrogenated solvent mixture containing less than 0.1 % by weight hydrogen peroxide. The hydrogenation also converts aldehyde and ketone by-products, such as acetaldehyde, to the corresponding alcohols, with the degree of conversion depending on the catalyst and the reaction conditions used. The conversion of the hydrogenation of acetaldehyde to ethanol can be adjusted by varying the reaction time and the hydrogen partial pressure and the temperature used in the catalytic hydrogenation and is preferably adjusted to provide a hydrogenated solvent mixture comprising from 1 to 1000 mg/kg of acetaldehyde.

In another preferred embodiment, which may be combined with the embodiments described in the two preceding paragraphs, a crude propene oxide comprising from 15 to 97 % by weight propene oxide and from 2 to 84 % by weight methanol is separated in step b) and this crude propene oxide is subjected to an extractive distillation in an extractive distillation column. An aqueous extraction solvent is used and a reactive compound containing an NH₂ group and capable of reacting with acetaldehyde at the conditions of the extractive distillation is fed to the extractive distillation column with a feed stream or separately at a feed point above the feed point for the crude propene oxide. The extractive distillation provides a purified propene oxide as an overhead product and a bottoms product comprising water and methanol. This bottoms product comprising water and methanol is subjected to a catalytic hydrogenation, and the hydrogenated bottoms product is passed to step c).

A crude propene oxide comprising from 15 to 97 % by weight propene oxide and from 2 to 84 % by weight methanol can be separated in step b) by the sequence of pressure reduction, distillation in a pre-separation column and propene stripping in a propene stripping column described further above. The extractive distillation of this crude propene oxide is carried out in an extractive distillation column. The extractive distillation column may be a tray column containing discrete trays such as sieve trays or bubble cap trays. The extractive distillation column may also be a packed column and both random packings as well as structured packings, such as metal gauze packings may be used. The extractive distillation column may also combine sections with discrete trays and sections with packings. The extractive distillation column will in general also comprise at least one overhead condenser and at least one column reboiler. The extractive distillation column preferably has at least two feed points, a feed point A for feeding the crude propene oxide in the middle section of the extractive distillation column and a feed point B for feeding aqueous extraction solvent located above feed point A. The feed points define three sections of the extractive distillation column, a stripping section between the column bottoms and feed point A, an extraction section between feed point A and feed point B and a rectifying section between feed point B and the top of the extractive distillation column. Preferably a distillation column is used that has a separation efficiency of 10 to 30 theoretical stages in the stripping section, a separation efficiency of 15 to 40 theoretical stages in the extraction section and a separation efficiency of 20 to 60 theoretical stages in the rectifying section, i.e. feed point B is preferably located from 15 to 40 theoretical separation stages above feed point A and from 20 to 60 theoretical separation stages below the top of the extractive distillation column.

The aqueous extraction solvent preferably comprises more than 80 % by weight water, more preferably more than 90 % by weight water. Preferably, the aqueous extraction solvent comprises no further solvent in addition to water. The extraction solvent is preferably fed in an amount providing a mass ratio of the extraction solvent relative to the amount of methanol contained in the crude propene oxide feed of from 0.01 to 1, more preferably from 0.03 to 0.2. The use of such an amount of aqueous extraction solvent provides effective extraction of methanol and a propene oxide product with a low content of methanol and at the same time avoids hydrolysis of propene oxide in the extractive distillation column.

In addition to the aqueous extraction solvent, a reactive compound containing an NH₂ group and capable of reacting with acetaldehyde at the conditions of the extractive distillation is fed to the extractive distillation column, either with a feed stream to the extraction column or separately at a feed point above the feed point for the crude propene oxide. The reactive compound is preferably fed to the extractive distillation column admixed with the extraction solvent. The amount of reactive compound fed to the distillation column is preferably chosen so that the molar ratio of the reactive compound relative to acetaldehyde is in the range of from 0.5 to 10. The use of such an amount of a reactive compound provides effective conversion of carbonyl compounds to high boiling compounds and provides a propene oxide product with a low content of acetaldehyde and other carbonyl compounds. At the same time, by-product formation by reactions of the reactive compound with propene oxide can be kept at a low level. In a preferred embodiment, the reactive compound has a structure R¹-Y-NH₂, where Y is oxygen or NR² and R¹ and R² independently of one another are hydrogen, an alkyl group or an aryl group. Salts of these reactive compounds with a protonated NH₂ group may be used as well. Preferred compounds of structure R¹-Y-NH₂ are hydrazine, hydrazine monohydrate, hydrazinium salts, hydroxylamine and hydroxylammonium salts. The amount of the reactive compound fed to the distillation column is then preferably chosen so that the molar ratio of the reactive compound relative to acetaldehyde is in the range of from 0.5 to 2. In an alternative preferred embodiment, the reactive compound is a diaminoalkane having from 2 to 6 carbon atoms, preferably 1,2-diaminoethane, 1,2-diaminopropane or 1,3-diaminopropane and most preferably 1,2-diaminoethane. The amount of reactive compound fed to the distillation column is then preferably chosen so that the molar ratio of the reactive compound relative to acetaldehyde is in the range of from 0.5 to 10, more preferably from 3 to 8. Compared to a reactive compound of structure R¹-Y-NH₂, the use of a diaminoalkane as reactive compound reduces the formation of volatile amines when reaction products resulting from the reaction of acetaldehyde with the reactive compound containing an NH₂ group are hydrogenated in the subsequent step of hydrogenating the bottoms product of the extractive distillation.

The bottoms product provided by the extractive distillation comprises water, methanol and reaction products formed by reaction of acetaldehyde with the reactive compound containing an NH₂ group. This bottoms product is subjected to catalytic hydrogenation for hydrogenating the reaction products resulting from the reaction of acetaldehyde with the reactive compound containing an NH₂ group. Oximes and hydrazones formed with reactive compound of structure R¹-Y-NH₂ will be hydrogenated with hydrogenolysis of the oxygen-nitrogen bond or the nitrogen-nitrogen bond. Imines formed from acetaldehyde and diaminoalkanes will be hydrogenated to the corresponding amines. The bottoms product provided by the extractive distillation is preferably combined with the solvent mixture separated in step b) before subjecting it to catalytic hydrogenation.

In the process of the invention, the bed of an acidic ion exchange resin used in step d) is periodically regenerated by passing a solution of a regenerating acid through the resin bed to provide a regenerated bed of ion exchange resin. The regenerated bed of ion exchange resin is then washed by passing methanol through the regenerated bed of ion exchange resin until the methanol exiting the resin bed has an apparent pH higher than 1.8 before the regenerated bed of ion exchange resin is reused in step d). According to the invention, the regenerated bed of ion exchange resin is washed until the methanol exiting the resin bed has an apparent pH higher than 2.0. The regenerated bed of ion exchange resin is preferably washed until the methanol exiting the resin bed has an apparent pH higher than 2.5 and more preferably higher than 3.0.

In a preferred embodiment the apparent pH of the reaction mixture passing the bed of catalyst is between 3.7 and 5.5, more preferred 3.9 to 4.5. Especially the apparent pH of the recycled methanol leaving the bed of ion exchange resin, before passing the catalyst in the reaction tube is maximum 8, more preferred maximum 6. A higher apparent pH might lead to a damage of the catalyst and a decrease of the yield of epoxidated propene.

The solution of a regenerating acid is preferably an aqueous solution. The regenerating acid may be a mineral acid, such as nitric acid, sulfuric acid, hydrochloric acid, phosphoric acid or perchloric acid; a sulfonic acids, such as methane sulfonic acid; or a carboxylic acid, such as formic acid, acetic acid, propionic acid, oxalic acid, malonic acid, succinic acid, maleic acid or fumaric acid. Preferred are sulfuric acid and phosphoric acid, most preferred is sulfuric acid.

Preferably, step d) of the process of the invention is carried out with down-flow through the bed of the acidic ion exchange resin and regenerating with the regenerating acid is carried out with up-flow of the solution of the regenerating acid through the bed of an acidic ion exchange resin. Changing flow direction for regeneration has the advantage of securing complete regeneration of the acidic ion exchange resin near the outlet for the treated methanol stream. Regeneration with up-flow can also be used for purging broken resin beads from the bed and prevents an increase in pressure drop across the bed of acidic ion exchange resin during long term operation.

In a preferred embodiment, regenerating the bed of an acidic ion exchange resin comprises the series of steps of
i) passing water through the resin bed to replace methanol,
ii) passing an aqueous solution of the regenerating acid through the resin bed, and,
iii) less than 24 h before reusing the regenerated bed of ion exchange resin in step d), passing methanol through the resin bed until the methanol exiting the resin bed has an apparent pH higher than 2.

Carrying out the final washing with methanol less than 24 h and preferably less than 12 h before the regenerated bed of ion exchange resin is reused in step d) reduces diffusion of acid entrapped within the ion exchange resin into the methanol remaining in the resin bed. Preferably, further steps of passing water through the resin bed followed by passing methanol through the resin bed to replace water are carried out between steps ii) and iii) to reduce the amount of methanol that has to be passed through the resin bed in step iii) for removing residual regenerating acid.

Preferably, methanol which exits the resin bed in the washing of the regenerated bed of acidic ion exchange resin is passed to step c).

### Examples

### Example 1 (comparative)

Propene was epoxidized in a pilot plant reactor in a reaction tube equipped with a cooling jacket and operated in trickle bed mode. A catalyst fixed bed of 10 I extruded titanium silicalite catalyst with cylindrical extrudates of 3.2 mm diameter and 3 mm length was arranged in the reaction tube. The pressure in the reactor was kept at 2.7 MPa by introducing nitrogen. Methanol was passed through the fixed bed for 2 h in down flow at a flow rate of 6 kg/h and a temperature of 20 °C. Recycling of methanol containing residual acid from regenerating an ion exchange bed was then imitated by passing methanol containing 250 ppm sulfuric acid through the catalyst fixed bed for 1 h having an apparent pH of 1.6, followed by passing acid free methanol through the catalyst fixed bed. After 9 h, an additional feeding of 96 g/h of a 1 % by weight solution of ammonia in methanol was started. After a further 3 h feeding of propene was started at a feed rate of 2.5 kg/h and after another 2 h feeding of a 70 % by weight aqueous solution of hydrogen peroxide was started at 0.7 kg/h. Feed rates for propene and hydrogen peroxide were then increased stepwise over a period of 4 h until a feed rate for propene of 4.6 kg/h and a feed rate for hydrogen peroxide of 1.37 kg/h were reached. Reaction was continued at these feed rates, adjusting the temperature during the epoxidation reaction to maintain an essentially constant conversion of hydrogen peroxide of 97.5 to 98 %. After 3 days of operation, a sudden increase in pressure drop along the catalyst bed was observed, indicating flooding of the catalyst bed, and the reaction had to be stopped.

The catalyst fixed bed was then washed by passing a mixture of 6 kg/h of methanol and 2.5 kg/h of propene through the fixed bed for 6 h followed by 6 kg/h of methanol only for 8 h, reducing the pressure in the reactor at a rate of 0.3 MPa/h until ambient pressure was reached. The catalyst was then withdrawn from the bottom of the reaction tube in 9 fractions and aliquots of 250 g of each fraction were air dried for 3 days followed by drying at 60 °C for 16 h in a drying cabinet. Aliquots of 35 g dried catalyst were then sieved for 10 s with a 2.5 mm test sieve on a test screening machine JEL 200 of J. Engelsmann AG. Table 1 shows the weight of fines with a size of less than 2.5 mm fraction for the three fractions from the top of the reaction tube (counting fractions from top downwards).

### Example 2

Example 1 was repeated without the step of passing methanol containing 250 ppm sulfuric acid through the catalyst fixed bed prior to starting the epoxidation reaction. The measured apparent pH was 4.1. Table 1 shows the weight of fines with a size of less than 2.5 mm for the three fractions from the top of the reaction tube. Further experiments at the same reaction conditions could be carried out for time periods of more than 60 days without a significant increase of pressure drop along the catalyst bed.

**Table 1 Weight of fines which had passed the 2.5 mm test sieve**

| Fraction | Example 1 | Example 2 |
|---|---|---|
| 1 | 0,19 g | 0,02 g |
| 2 | 0,19 g | 0,09 g |
| 3 | 0,16 g | 0,09 g |

### Example 3

In a production plant for producing propene oxide with a tube bundle reactor containing fixed beds of extruded titanium silicalite in the tubes and a methanol recycle, where the recovered methanol was passed through beds of an acidic ion exchange resin, an incident, where methanol from a regenerated and insufficiently washed ion exchange resin was passed to the reactor, wherein the apparent pH was 1.88, led to a sudden increase in pressure drop along the reactor similar to the increase observed in example 1. Regenerating the acidic ion exchange resin with the washing step of the invention prevented such sudden increase in pressure drop during extended operation over a period of several thousand hours of operating the plant.

### Example 4

Propene was epoxidized in a production plant reactor. A catalyst fixed bed of extruded titanium silicalite catalyst with cylindrical extrudates was arranged in reaction tubes of a tube bundle reactor, wherein a reaction mixture of propene with hydrogen peroxide in the presence of a methanol solvent was reacted. The solvent mixture was separated from crude propene oxide and purified in a distillation procedure, providing a recovered methanol as an overhead product. The recovered methanol was passed over a bed of an acidic ion exchange resin, especially a polystyrene based sulfonic acid resin. The recycled methanol was passed to a reaction mixture reacting in the reaction tube. The apparent pH of the recycled methanol was measured constantly after passing over the acidic ion exchange resin, before entering the reaction tubes. The state of the catalyst, especially the state active or damaged, was observed by monitoring of the pressure drop and temperature within the tube bundle reactor. A significant increase of pressure drop and a sudden temperature change along the catalyst bed can be observed when the catalyst is damaged.

The bed of an acidic ion exchange resin was periodically regenerated by passing a solution of a regenerating acid, especially sulfuric acid, through said resin bed to provide a regenerated bed of ion exchange resin and said regenerated bed of ion exchange resin was washed by passing methanol through said regenerated bed of ion exchange resin. After the start of operation of a regenerated bed of ion exchange resin the recycled methanol leaving the bed of ion exchange resin may be more acidic than in steady state operation. This could lead to a damage of the catalyst. The apparent pH was measured after leaving an ion exchange resin bed leading to a damage or no damage of the catalyst as shown in Table.

**Table 2 pH dependent effect on state of epoxidation catalyst in production plant**

| Measurement | Apparent pH | State of epoxidation catalyst |
|---|---|---|
| 1 (comparative) | 1,88 | Damage |
| 2 | 2,14 | No damage |
| 3 | 3,15 | No damage |
| 4 | 3,94 | No damage |

## Claims

1. A process for the epoxidation of propene comprising the steps
a) reacting propene with hydrogen peroxide in the presence of a methanol solvent and a shaped titanium zeolite epoxidation catalyst in a fixed bed reactor to provide a reaction mixture,
b) separating from the reaction mixture of step a) a crude propene oxide and a solvent mixture comprising methanol and water,
c) separating the solvent mixture of step b) in at least one distillation stage, providing a recovered methanol as an overhead product,
d) passing the recovered methanol of step c) through a bed of an acidic ion exchange resin, providing a treated methanol, and
e) recycling the treated methanol of step d) as methanol solvent to step a), wherein said bed of an acidic ion exchange resin is periodically regenerated by passing a solution of a regenerating acid through said resin bed to provide a regenerated bed of ion exchange resin and said regenerated bed of ion exchange resin is washed by passing methanol through said regenerated bed of ion exchange resin until the methanol exiting the resin bed has an apparent pH higher than 2.0 before the regenerated bed of ion exchange resin is reused in step d).

2. The process of claim 1 , wherein the methanol exiting the resin bed has an apparent pH higher than 2.5.

3. The process of one of the claims 1 and 2, wherein regenerating the bed of an acidic ion exchange resin comprises the series of steps of
i) passing water through the resin bed to replace methanol,
ii) passing an aqueous solution of said regenerating acid through the resin bed, and,
iii) less than 24 h before reusing the regenerated bed of ion exchange resin in step d), passing methanol through the resin bed until the methanol exiting the resin bed has an apparent pH higher than 2.

4. The process of claim 3, further comprising the steps of passing water through the resin bed followed by passing methanol through the resin bed to replace water between steps ii) and iii).

5. The process of any one of claims 1 to 4, wherein step d) is carried out with down-flow through the bed of an acidic ion exchange resin and regenerating with the regenerating acid is carried out with up-flow through the bed of an acidic ion exchange resin.

6. The process of any one of claims 1 to 5, wherein methanol, which exits the resin bed in the washing of the regenerated bed of acidic ion exchange resin, is passed to step c).

7. The process of any one of claims 1 to 6, wherein sulfuric acid is used as the regenerating acid.

8. The process of any one of claims 1 to 7, wherein the acidic ion exchange resin used in step e) comprises sulfonic acid groups.

9. The process of any one of claims 1 to 8, wherein the apparent pH of the treated methanol obtained in step d) is monitored and the acidic ion exchange resin is regenerated when the apparent pH of the treated methanol exceeds a threshold value selected in the range of from 4 to 7.

10. The process of any one of claims 1 to 9, wherein ammonia is added in step a) with a weight ratio of ammonia to the initial amount of hydrogen peroxide of from 0.0001 to 0.003.

11. The process of any one of claims 1 to 10, wherein the solvent mixture separated in step b) is subjected to a catalytic hydrogenation before it is separated in step c).

12. The process of any one of claims 1 to 11, wherein the crude propene oxide separated in step b) comprises from 15 to 97 % by weight propene oxide and from 2 to 84 % by weight methanol and is subjected to an extractive distillation in an extractive distillation column, using an aqueous extraction solvent and feeding a reactive compound, containing an NH₂ group and capable of reacting with acetaldehyde at the conditions of said extractive distillation, to said extractive distillation column with a feed stream to the extractive distillation column or separately at a feed point above the feed point for the crude propene oxide, providing a purified propene oxide as an overhead product and a bottoms product comprising water and methanol; said bottoms product comprising water and methanol is subjected to a catalytic hydrogenation, and the hydrogenated bottoms product is passed to step c).

13. The process of claim 12, wherein the reactive compound is selected from hydrazine, hydrazine monohydrate, hydrazinium salts, hydroxylamine, hydroxylammonium salts and diaminoalkanes having from 2 to 6 carbon atoms.

14. The process of any one of claims 1 to 13, wherein an acid is added to at least one distillation stage of step c) or to the solvent mixture obtained in step b) before separating it in step c).

15. The process of any one of claims 1 to 14, wherein steps a) to e) are carried out continuously.

## Patentansprüche

1. Verfahren zur Epoxidierung von Propen, umfassend die Schritte
a) Umsetzen von Propen mit Wasserstoffperoxid in Gegenwart eines Methanol-Lösungsmittels und eines geformten Titanzeolith-Epoxidierungskatalysators in einem Festbettreaktor zur Bereitstellung einer Reaktionsmischung,
b) Abtrennen eines rohen Propenoxids und eines Lösungsmittelgemischs, das Methanol und Wasser umfasst, aus der Reaktionsmischung aus Schritt a),
c) Trennen des Lösungsmittelgemischs aus Schritt b) in mindestens einer Destillationsstufe, wobei ein zurückgewonnenes Methanol als Kopfprodukt bereitgestellt wird,
d) Leiten des zurückgewonnenen Methanols aus Schritt c) durch ein Bett eines sauren Ionenaustauscherharzes, wobei ein behandeltes Methanol bereitgestellt wird, und
e) Zurückführen des behandelten Methanols aus Schritt d) als Methanol-Lösungsmittel zu Schritt a), wobei das Bett eines sauren Ionenaustauscherharzes periodisch regeneriert wird, indem eine Lösung einer regenerierenden Säure durch das Harzbett geleitet wird, wodurch ein regeneriertes Bett von Ionenaustauscherharz bereitgestellt wird, und das regenerierte Bett von Ionenaustauscherharz gewaschen wird, indem Methanol durch das regenerierte Bett aus Ionenaustauscherharz geleitet wird, bis das aus dem Harzbett austretende Methanol einen scheinbaren pH-Wert von mehr als 2,0 aufweist, bevor das regenerierte Bett von Ionenaustauscherharz in Schritt d) wiederverwendet wird.

2. Verfahren nach Anspruch 1, wobei das aus dem Harzbett austretende Methanol einen scheinbaren pH-Wert von mehr als 2,5 aufweist.

3. Verfahren nach einem der Ansprüche 1 und 2, wobei das Regenerieren des Betts eines sauren Ionenaustauscherharzes die folgende Abfolge von Schritten umfasst:
i) Leiten von Wasser durch das Harzbett zum Ersatz von Methanol,
ii) Leiten einer wässrigen Lösung der regenerierenden Säure durch das Harzbett und
iii) weniger als 24 h vor der Wiederverwendung des regenerierten Betts von Ionenaustauscherharz in Schritt d), Leiten von Methanol durch das Harzbett, bis das aus dem Harzbett austretende Methanol einen scheinbaren pH-Wert von mehr als 2 aufweist.

4. Verfahren nach Anspruch 3, ferner umfassend die Schritte des Leitens von Wasser durch das Harzbett und des anschließenden Leitens von Methanol durch das Harzbett zum Ersatz von Wasser zwischen den Schritten ii) und iii).

5. Verfahren nach einem der Ansprüche 1 bis 4, wobei Schritt d) im Abwärtsstrom durch das Bett eines sauren Ionenaustauscherharzes durchgeführt wird und die Regenerierung mit der regenerierenden Säure im Aufwärtsstrom durch das Bett eines sauren Ionenaustauscherharzes durchgeführt wird.

6. Verfahren nach einem der Ansprüche 1 bis 5, wobei Methanol, das beim Waschen des regenerierten Betts von saurem Ionenaustauscherharz aus dem Harzbett austritt, zu Schritt c) geleitet wird.

7. Verfahren nach einem der Ansprüche 1 bis 6, wobei Schwefelsäure als regenerierende Säure verwendet wird.

8. Verfahren nach einem der Ansprüche 1 bis 7, wobei das in Schritt e) verwendete saure Ionenaustauscherharz Sulfonsäuregruppen umfasst.

9. Verfahren nach einem der Ansprüche 1 bis 8, wobei der scheinbare pH-Wert des in Schritt d) erhaltenen behandelten Methanols überwacht wird und das saure Ionenaustauscherharz regeneriert wird, wenn der scheinbare pH-Wert des behandelten Methanols einen im Bereich von 4 bis 7 gewählten Schwellenwert überschreitet.

10. Verfahren nach einem der Ansprüche 1 bis 9, wobei Ammoniak in Schritt a) mit einem Gewichtsverhältnis von Ammoniak zur Anfangsmenge an Wasserstoffperoxid von 0,0001 bis 0,003 zugegeben wird.

11. Verfahren nach einem der Ansprüche 1 bis 10, wobei das in Schritt b) abgetrennte Lösungsmittelgemisch einer katalytischen Hydrierung unterworfen wird, bevor es in Schritt c) getrennt wird.

12. Verfahren nach einem der Ansprüche 1 bis 11, wobei das in Schritt b) abgetrennte rohe Propenoxid 15 bis 97 Gew.-% Propenoxid und 2 bis 84 Gew.-% Methanol umfasst und einer Extraktivdestillation in einer Extraktionsdestillationskolonne unter Verwendung eines wässrigen Extraktionslösungsmittels und Einspeisung einer reaktiven Verbindung, die eine NH₂-Gruppe enthält und unter den Bedingungen der Extraktivdestillation mit Acetaldehyd reagieren kann, zu der Extraktionsdestillationskolonne mit einem Einsatzstrom in die Extraktionsdestillationskolonne oder separat an einem Einspeisepunkt oberhalb des Einspeisepunkts für das rohe Propenoxid unterworfen wird, wobei ein gereinigtes Propenoxid als Kopfprodukt und ein Wasser und Methanol umfassendes Sumpfprodukt bereitgestellt wird; wobei das Wasser und Methanol umfassende Sumpfprodukt einer katalytischen Hydrierung unterworfen wird und das hydrierte Sumpfprodukt zu Schritt c) geleitet wird.

13. Verfahren nach Anspruch 12, wobei die reaktive Verbindung aus Hydrazin, Hydrazinmonohydrat, Hydraziniumsalzen, Hydroxylamin, Hydroxylammoniumsalzen und Diaminoalkanen mit 2 bis 6 Kohlenstoffatomen ausgewählt wird.

14. Verfahren nach einem der Ansprüche 1 bis 13, wobei eine Säure zu mindestens einer Destillationsstufe von Schritt c) oder zu dem in Schritt b) erhaltenen Lösungsmittelgemisch, bevor es in Schritt c) getrennt wird, gegeben wird.

15. Verfahren nach einem der Ansprüche 1 bis 14, wobei die Schritte a) bis e) kontinuierlich durchgeführt werden.

## Revendications

1. Procédé d'époxydation de propène comprenant les étapes
a) mise en réaction de propène avec du peroxyde d'hydrogène en présence d'un solvant de méthanol et d'un catalyseur d'époxydation de zéolite de titane façonné dans un réacteur à lit fixe pour obtenir un mélange réactionnel,
b) séparation depuis le mélange réactionnel de l'étape a) d'un oxyde de propène brut et d'un mélange de solvants comprenant du méthanol et de l'eau,
c) séparation du mélange de solvants de l'étape b) dans au moins une étape de distillation, fournissant un méthanol récupéré comme un produit de tête,
d) passage du méthanol récupéré de l'étape c) à travers un lit de résine échangeuse d'ions acide, fournissant un méthanol traité, et
e) recyclage du méthanol traité de l'étape d) comme solvant de méthanol à l'étape a), dans lequel ledit lit de résine échangeuse d'ions acide est périodiquement régénéré en faisant passer une solution d'un acide régénérant à travers ledit lit de résine pour fournir un lit régénéré de résine échangeuse d'ions et ledit lit régénéré de résine échangeuse d'ions est lavé en faisant passer du méthanol à travers ledit lit régénéré de résine échangeuse d'ions jusqu'à ce que le méthanol sortant du lit de résine ait un pH apparent supérieur à 2,0 avant que le lit régénéré de résine échangeuse d'ions ne soit réutilisé dans l'étape d).

2. Procédé selon la revendication 1, dans lequel le méthanol sortant du lit de résine a un pH apparent supérieur à 2,5.

3. Procédé selon l'une des revendications 1 et 2, dans lequel la régénération du lit d'une résine échangeuse d'ions acide comprend la série d'étapes de
i) passage d'eau à travers le lit de résine pour remplacer le méthanol,
ii) passage d'une solution aqueuse dudit acide régénérant à travers le lit de résine, et,
iii) moins de 24 h avant la réutilisation du lit régénéré de résine échangeuse d'ions à l'étape d), passage du méthanol à travers le lit de résine jusqu'à ce que le méthanol sortant du lit de résine ait un pH apparent supérieur à 2.

4. Procédé selon la revendication 3, comprenant en outre les étapes de passage d'eau à travers le lit de résine, puis de passage de méthanol à travers le lit de résine pour remplacer l'eau entre les étapes ii) et iii).

5. Procédé selon l'une quelconque des revendications 1 à 4, dans lequel l'étape d) est effectuée avec un flux descendant à travers le lit d'une résine échangeuse d'ions acide et la régénération avec l'acide régénérant est effectuée avec un flux ascendant à travers le lit d'une résine échangeuse d'ions acide.

6. Procédé selon l'une quelconque des revendications 1 à 5, dans lequel le méthanol, qui sort du lit de résine lors du lavage du lit régénéré de résine échangeuse d'ions acide, est passé à l'étape c).

7. Procédé selon l'une quelconque des revendications 1 à 6, dans lequel l'acide sulfurique est utilisé comme l'acide régénérant.

8. Procédé selon l'une quelconque des revendications 1 à 7, dans lequel la résine échangeuse d'ions acide utilisée dans l'étape e) comprend des groupes acide sulfonique.

9. Procédé selon l'une quelconque des revendications 1 à 8, dans lequel le pH apparent du méthanol traité obtenu dans l'étape d) est surveillé et la résine échangeuse d'ions acide est régénérée lorsque le pH apparent du méthanol traité dépasse une valeur seuil choisie dans la plage de 4 à 7.

10. Procédé selon l'une quelconque des revendications 1 à 9, dans lequel de l'ammoniac est ajouté dans l'étape a) avec un rapport pondéral d'ammoniac sur la quantité initiale de peroxyde d'hydrogène de 0,0001 à 0,003.

11. Procédé selon l'une quelconque des revendications 1 à 10, dans lequel le mélange de solvants séparé dans l'étape b) est soumis à une hydrogénation catalytique avant d'être séparé dans l'étape c).

12. Procédé selon l'une quelconque des revendications 1 à 11, dans lequel l'oxyde de propène brut séparé dans l'étape b) comprend de 15 à 97 % en poids d'oxyde de propène et de 2 à 84 % en poids de méthanol et est soumis à une distillation extractive dans une colonne de distillation extractive, en utilisant un solvant d'extraction aqueux et en introduisant un composé réactif, contenant un groupe NH₂ et capable de réagir avec l'acétaldéhyde dans les conditions de ladite distillation extractive, dans ladite colonne de distillation extractive avec un flux d'alimentation vers la colonne de distillation extractive ou séparément en un point d'alimentation au-dessus du point d'alimentation pour l'oxyde de propène brut, fournissant un oxyde de propène purifié comme produit de tête et un produit de queue comprenant de l'eau et du méthanol ; ledit produit de queue comprenant de l'eau et du méthanol est soumis à une hydrogénation catalytique, et le produit de queue hydrogéné est passé à l'étape c).

13. Procédé selon la revendication 12, dans lequel le composé réactif est choisi parmi l'hydrazine, le monohydrate d'hydrazine, les sels d'hydrazinium, l'hydroxylamine, les sels d'hydroxylammonium et les diaminoalcanes ayant de 2 à 6 atomes de carbone.

14. Procédé selon l'une quelconque des revendications 1 à 13, dans lequel un acide est ajouté à au moins une étape de distillation de l'étape c) ou au mélange de solvants obtenu dans l'étape b) avant sa séparation dans l'étape c).

15. Procédé selon l'une quelconque des revendications 1 à 14, dans lequel les étapes a) à e) sont effectuées de manière continue.
